# EUROPEAN PATENT APPLICATION

(11) **EP 4 821 421 A1**
(43) Date of publication of application: **14.10.2026**
(21) Application number: 24903415.8
(22) Date of filing: 12.11.2024
(51) Int. Cl.: A61B 1/00

(54) **CATHETER FOR DIAGNOSTIC IMAGING**

(30) Priority: 11.12.2023 JP 2023208858
(71) Applicant: Terumo Kabushiki Kaisha, Tokyo 151-0072 (JP)
(72) Inventor: TAJIMA, Chihiro, Ashigarakami-gun, Kanagawa 259-0151 (JP); SUGIHARA, Soichiro, Fujinomiya-shi, Shizuoka 418-0015 (JP); TANI, Kentaro, Fujinomiya-shi, Shizuoka 418-0015 (JP); WU, Xiaobin, Ashigarakami-gun, Kanagawa 259-0151 (JP)
(74) Representative: Casalonga
(86) International application number: PCT/JP2024/040218
(87) International publication number: WO 2025/126755

(57) **Abstract**

An image diagnosis catheter includes: a proximal outer tube; a proximal inner tube; an outer tube support tube; and a drive shaft, wherein, in a fully advanced state in which the proximal inner tube is pushed farthest into the proximal outer tube, the outer tube support tube, the proximal inner tube, and the proximal outer tube form a multiple tube structure located in an order of the outer tube support tube, the proximal inner tube, and the proximal outer tube toward a radially outer side from the drive shaft, in a fully retracted state in which the proximal inner tube is drawn out farthest from the proximal outer tube, a rear end of the outer tube support tube is located on a front side of a front end of the proximal inner tube, an outer peripheral surface of a front end portion of the proximal inner tube includes an enlarged-diameter portion having a rear end portion as a stopper that abuts against the proximal outer tube when the proximal inner tube is drawn out from the proximal outer tube and reaches the fully retracted state, an inner peripheral surface of the front end portion of the proximal inner tube includes a guide surface extending rearward while gradually decreasing in diameter from a front end surface of the proximal inner tube, and the guide surface is configured to guide a rear end portion of the outer tube support tube when the proximal inner tube is pushed into the proximal outer tube from the fully retracted state.

## Description

### Technical Field

The present disclosure relates to an image diagnosis catheter.

### Background Art

A known image diagnosis catheter includes a proximal outer tube, a proximal inner tube, an outer tube support tube, and a drive shaft, and forms a multiple tube structure in which, in a fully advanced state in which the proximal inner tube is pushed farthest into the proximal outer tube, the outer tube support tube, the proximal inner tube, and the proximal outer tube are located in this order toward a radially outer side from the drive shaft (see, for example, Patent Literature 1).

### Citation List

### Patent Literature

Patent Literature 1: WO 2023/008539 A

### Summary of Invention

### Technical Problem

The image diagnosis catheter as described above may have a configuration in which, in consideration of priming performance and the like, a rear end of the outer tube support tube is located on a front side of a front end of the proximal inner tube in a fully retracted state in which the proximal inner tube is drawn out farthest from the proximal outer tube. In this case, when the proximal inner tube is pushed into the proximal outer tube from the fully retracted state, if the position of the rear end of the outer tube support tube becomes eccentric relative to the position of the front end of the proximal inner tube due to an external force and a rear end surface of the outer tube support tube comes into contact with a front end surface of the proximal inner tube, the rear end of the outer tube support tube may not be smoothly accommodated inside the proximal inner tube.

Accordingly, an object of the present disclosure is to provide an image diagnosis catheter in which a rear end of an outer tube support tube is smoothly accommodated inside a proximal inner tube when the proximal inner tube is pushed into a proximal outer tube from a fully retracted state.

### Solution to Problem

One aspect of the present disclosure is as follows.

[1] An image diagnosis catheter including: a proximal outer tube; a proximal inner tube; an outer tube support tube; and a drive shaft,
   wherein, in a fully advanced state in which the proximal inner tube is pushed farthest into the proximal outer tube, the outer tube support tube, the proximal inner tube, and the proximal outer tube form a multiple tube structure located in an order of the outer tube support tube, the proximal inner tube, and the proximal outer tube toward a radially outer side from the drive shaft,
   in a fully retracted state in which the proximal inner tube is drawn out farthest from the proximal outer tube, a rear end of the outer tube support tube is located on a front side of a front end of the proximal inner tube,
   an outer peripheral surface of a front end portion of the proximal inner tube includes an enlarged-diameter portion having a rear end portion as a stopper that abuts against the proximal outer tube when the proximal inner tube is drawn out from the proximal outer tube and reaches the fully retracted state,
   an inner peripheral surface of the front end portion of the proximal inner tube includes a guide surface extending rearward while gradually decreasing in diameter from a front end surface of the proximal inner tube, and
   the guide surface is configured to guide a rear end portion of the outer tube support tube when the proximal inner tube is pushed into the proximal outer tube from the fully retracted state.
[2] The image diagnosis catheter according to [1], wherein an outer diameter of the enlarged-diameter portion is constant from a front end portion to the rear end portion.
[3] The image diagnosis catheter according to [1] or [2], including:
   an inner tube support tube,
   wherein, in the fully advanced state, the inner tube support tube, the outer tube support tube, the proximal inner tube, and the proximal outer tube form the multiple tube structure located in an order of the inner tube support tube, the outer tube support tube, the proximal inner tube, and the proximal outer tube toward the radially outer side from the drive shaft, and
   in the fully retracted state, a front end of the inner tube support tube is located on the front side of the rear end of the outer tube support tube.
[4] The image diagnosis catheter according to [3], wherein a telescopic mechanism formed of the proximal outer tube, the proximal inner tube, the outer tube support tube, and the inner tube support tube includes an outer flow path formed between the proximal inner tube and the proximal outer tube, and the inner tube support tube and the outer tube support tube in the fully retracted state, and the outer flow path allows priming liquid to flow from a rear end portion of the telescopic mechanism to a front end portion of the telescopic mechanism in the fully retracted state.
[5] An image diagnosis catheter manufacturing method for manufacturing the image diagnosis catheter according to any one of [1] to [4], the method including:
   a proximal inner tube forming step of forming the guide surface and the enlarged-diameter portion by using a mold including a guide surface molding surface having a shape along the guide surface and an enlarged-diameter portion molding surface having a shape along an outer peripheral surface of the enlarged-diameter portion, and bringing a front end portion in a molten state of a tubular body that is a preformed product of the proximal inner tube into contact with the guide surface molding surface and the enlarged-diameter portion molding surface to deform the front end portion.
[6] The image diagnosis catheter manufacturing method according to [5], wherein the enlarged-diameter portion molding surface has a constant diameter from a front end portion to a rear end portion, an outer diameter of the tubular body is smaller than the diameter of the enlarged-diameter portion molding surface, and a diameter of a front end of the guide surface molding surface extending rearward while gradually decreasing in diameter is larger than an inner diameter of a front end portion of the tubular body.

### Advantageous Effects of Invention

According to the present disclosure, an image diagnosis catheter can be provided in which a rear end of an outer tube support tube is smoothly accommodated inside a proximal inner tube when the proximal inner tube is pushed into a proximal outer tube from a fully retracted state.

### Brief Description of Drawings

[Fig. 1] Fig. 1 is an external view showing a fully advanced state of the image diagnosis catheter according to one embodiment of the present disclosure.
[Fig. 2] Fig. 2 is an external view showing a fully retracted state of the image diagnosis catheter of Fig. 1.
[Fig. 3] Fig. 3 is a partially enlarged cross-sectional view of Fig. 1.
[Fig. 4] Fig. 4 is a partially enlarged cross-sectional view of Fig. 2.
[Fig. 5A] Fig. 5A is a cross-sectional view showing a state when an end portion of a tubular body, which is a preformed product of the proximal inner tube, is brought into contact with a mold in order to manufacture the image diagnosis catheter of Fig. 1.
[Fig. 5B] Fig. 5B is a cross-sectional view showing a state when deformation of the end portion of the tubular body by a guide surface molding surface of the mold is started from the state of Fig. 5A.
[Fig. 5C] Fig. 5C is a cross-sectional view showing a state when deformation of the end portion of the tubular body by an enlarged-diameter portion molding surface of the mold is started from the state of Fig. 5B.
[Fig. 5D] Fig. 5D is a cross-sectional view showing a state when deformation of the end portion of the tubular body by the guide surface molding surface and the enlarged-diameter portion molding surface is completed from the state of Fig. 5C.

### Description of Embodiments

Hereinafter, an embodiment of the present disclosure will be described in detail by way of example with reference to the drawings.

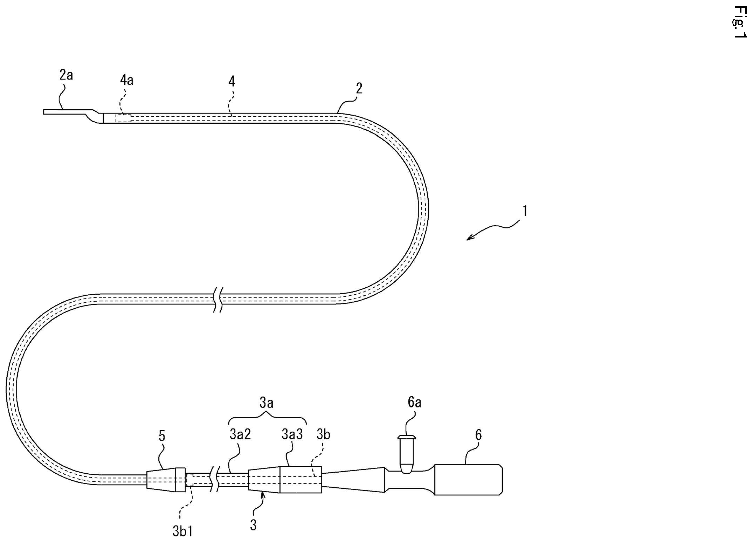

As shown in Figs. 1 to 2, an image diagnosis catheter 1 according to one embodiment of the present disclosure includes a sheath 2 inserted into a body lumen such as a vessel of a living body such as a patient (a blood vessel such as a coronary artery), a telescopic mechanism 3 formed of a proximal outer tube 3a and a proximal inner tube 3b, and a drive shaft 4 having a signal transmitter and receiver 4a at a distal end portion, and, in a state in which the sheath 2 is inserted into the body lumen, generates a tomographic image of a peripheral portion of the body lumen for diagnosis by the signal transmitter and receiver 4a while moving the drive shaft 4 toward the proximal side in the sheath 2 by the telescopic mechanism 3. The signal transmitter and receiver 4a may have a structure of an intravascular ultrasound (IVUS) system, a structure of an optical coherence tomography (OCT) system, or a structure including both.

The image diagnosis catheter 1 may have a configuration in which the image diagnosis catheter 1 is connected to an external device (not shown), and the external device rotates the drive shaft 4 in the sheath 2 and pulls the proximal inner tube 3b toward the proximal side while controlling transmission and reception of signals by the signal transmitter and receiver 4a with respect to a peripheral portion of the body lumen, thereby generating a tomographic image. A distal end portion of the sheath 2 may have a guide wire insertion member 2a through which a guide wire (not shown) passes.

The telescopic mechanism 3 moves the drive shaft 4 toward the proximal side in the sheath 2 by the proximal inner tube 3b being pulled toward the proximal side relative to the proximal outer tube 3a and extending.

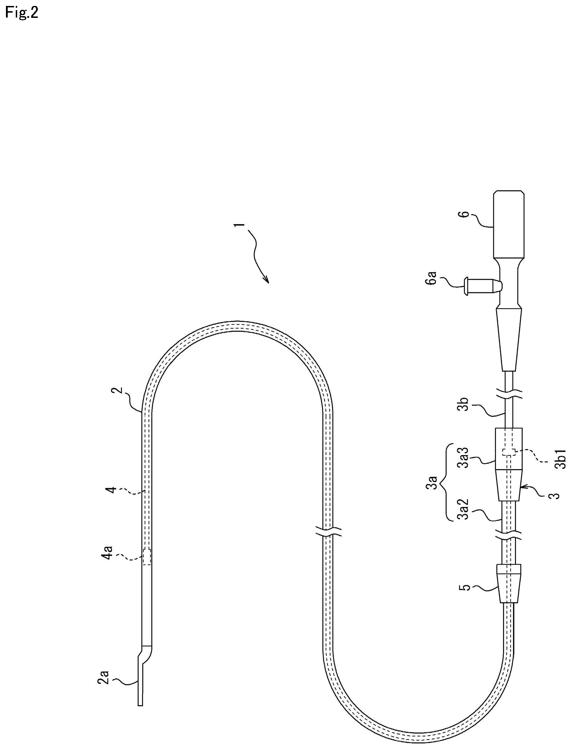

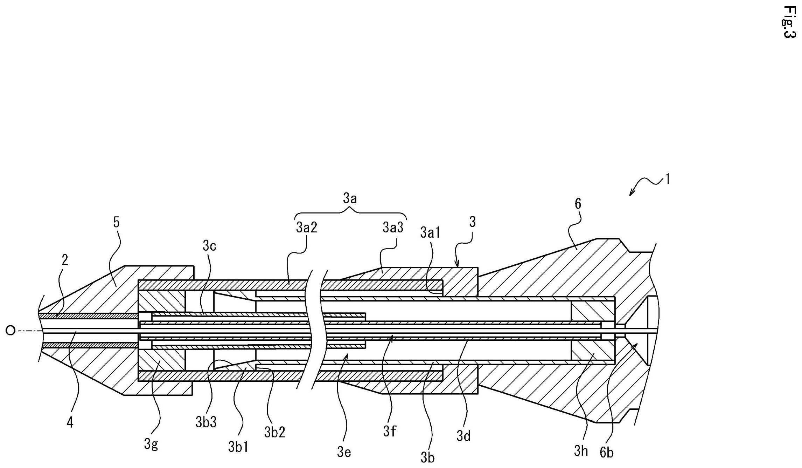

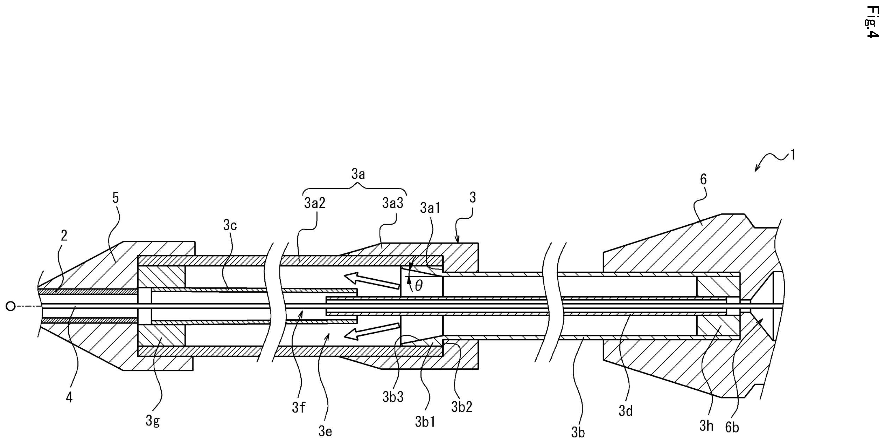

More specifically, as shown in Figs. 1 to 4, the image diagnosis catheter 1 includes the proximal outer tube 3a, the proximal inner tube 3b, the outer tube support tube 3c, and the drive shaft 4, the outer tube support tube 3c, the proximal inner tube 3b, and the proximal outer tube 3a form a multiple tube structure located in this order toward a radially outer side from the drive shaft 4 in a fully advanced state in which the proximal inner tube 3b is pushed farthest into the proximal outer tube 3a (see Figs. 1 and 3), a rear end of the outer tube support tube 3c is located on a front side of a front end of the proximal inner tube 3b in a fully retracted state in which the proximal inner tube 3b is drawn out farthest from the proximal outer tube 3a (see Figs. 2 and 4), an outer peripheral surface of a front end portion of the proximal inner tube 3b has an enlarged-diameter portion 3b1 having a rear end portion as a stopper 3b2 that abuts against the proximal outer tube 3a when the proximal inner tube 3b is drawn out from the proximal outer tube 3a and reaches the fully retracted state, an inner peripheral surface of the front end portion of the proximal inner tube 3b has a guide surface 3b3 extending rearward while gradually decreasing in diameter from a front end surface of the proximal inner tube 3b, and the guide surface 3b3 is configured to guide a rear end portion of the outer tube support tube 3c when the proximal inner tube 3b is pushed into the proximal outer tube 3a from the fully retracted state.

In the present embodiment, a front-rear direction is a direction along a central axis O of the proximal inner tube 3b, a front side is a distal side (patient side) of the image diagnosis catheter 1, a rear side is a proximal side (operator side), a radial direction is a direction perpendicular to the central axis O, and a circumferential direction is a direction around the central axis O.

According to the above configuration, an inner diameter of the front end of the proximal inner tube 3b can be increased by an amount corresponding to provision of the guide surface 3b3, and therefore contact of the rear end surface of the outer tube support tube 3c with the front end surface of the proximal inner tube 3b can be suppressed when the proximal inner tube 3b is pushed into the proximal outer tube 3a from the fully retracted state, and the rear end portion of the outer tube support tube 3c can be guided to an inside of the proximal inner tube 3b (a radially inner side and a rear side) by the guide surface 3b3 extending rearward while gradually decreasing in diameter from the front end surface of the proximal inner tube 3b. Accordingly, the image diagnosis catheter 1 can be realized in which the rear end of the outer tube support tube 3c is smoothly accommodated inside the proximal inner tube 3b when the proximal inner tube 3b is pushed into the proximal outer tube 3a from the fully retracted state. In the present embodiment, the guide surface 3b3 extends linearly rearward while gradually decreasing in diameter, but the guide surface 3b3 is not limited thereto, and, for example, the guide surface 3b3 may have a configuration extending rearward while gradually decreasing in diameter in a bent-line shape, a curved shape, or a stepped shape.

In the present embodiment, as described above, the telescopic mechanism 3 is provided in an orientation in which the proximal inner tube 3b is pulled toward the proximal side relative to the proximal outer tube 3a, but the telescopic mechanism 3 may also be provided in an opposite orientation. In this case, the telescopic mechanism 3 has a configuration in which the proximal outer tube 3a is pulled toward the proximal side relative to the proximal inner tube 3b and thereby extends, so as to move the drive shaft 4 toward the proximal side in the sheath 2, and the front side becomes the proximal side and the rear side becomes the distal side.

An outer diameter of the enlarged-diameter portion 3b1 is constant from a front end portion to a rear end portion. Here, "constant" means being constant to an extent that allows a slight inclination due to a draft angle normally provided in a mold when the enlarged-diameter portion 3b1 is formed by molding. According to the above configuration, strength of the stopper 3b2 (the rear end portion of the enlarged-diameter portion 3b1) can be easily ensured.

The image diagnosis catheter 1 has an inner tube support tube 3d, and, in the fully advanced state, the inner tube support tube 3d, the outer tube support tube 3c, the proximal inner tube 3b, and the proximal outer tube 3a form a multiple tube structure in which the inner tube support tube 3d, the outer tube support tube 3c, the proximal inner tube 3b, and the proximal outer tube 3a are located in this order toward a radially outer side from the drive shaft 4, and, in the fully retracted state, a front end of the inner tube support tube 3d is located on a front side of a rear end of the outer tube support tube 3c. According to the above configuration, when the proximal inner tube 3b is pushed into the proximal outer tube 3a from the fully retracted state, the inner tube support tube 3d can suppress eccentricity of the position of the rear end of the outer tube support tube 3c relative to the position of the front end of the proximal inner tube 3b due to an external force. Therefore, the rear end of the outer tube support tube 3c can be more easily smoothly accommodated inside the proximal inner tube 3b.

Dimensions of the guide surface 3b3 of the proximal inner tube 3b and an inclination angle θ can be set as appropriate, but a diameter at a front end of the guide surface 3b3 is preferably 1.75 mm or more, a length of the guide surface 3b3 in the front-rear direction is preferably 1.8 mm or more, and the inclination angle θ relative to the central axis O is preferably 9 degrees or more.

In the present embodiment, a front end portion of the proximal inner tube 3b has a front end surface extending radially outward from the front end of the guide surface 3b3, and the front end surface extends linearly in the radial direction, but the present disclosure is not limited thereto, and, for example, the front end surface may have a configuration extending in a curved shape, or a configuration may be adopted in which the front end of the guide surface 3b3 is directly continuous with the front end of the enlarged-diameter portion 3b1 without providing the front end surface.

A rear end portion of an inner peripheral surface of the proximal outer tube 3a has a step surface 3a1 that decreases in diameter in a stepped manner and faces frontward, and the proximal inner tube 3b stops when the stopper 3b2 abuts against the step surface 3a1 when the proximal inner tube 3b reaches the fully retracted state. The proximal outer tube 3a has a cylindrical proximal outer tube body 3a2 and a unit connector 3a3 connected to a rear end portion of the proximal outer tube body 3a2, and the unit connector 3a3 has the step surface 3a1 against which the stopper 3b2 abuts.

The telescopic mechanism 3 has an outer flow path 3e formed between the proximal inner tube 3b and the proximal outer tube 3a, and the inner tube support tube 3d and the outer tube support tube 3c in the fully retracted state, and the outer flow path 3e allows priming liquid such as saline to flow from a rear end portion of the telescopic mechanism 3 to a front end portion of the telescopic mechanism 3 in the fully retracted state. Further, in the telescopic mechanism 3, since the rear end of the outer tube support tube 3c is located on a front side of the front end of the proximal inner tube 3b in the fully retracted state, a large flow-path cross-sectional area of the outer flow path 3e in the proximal inner tube 3b can be ensured when priming is performed in the fully retracted state, and smooth priming can therefore be achieved (see the white outlined arrows in Fig. 4).

If the drive shaft 4 buckles in the telescopic mechanism 3 when the state is changed from the fully retracted state to the fully advanced state after priming, damage such as the drive shaft 4 being twisted off may occur, for example, when the drive shaft 4 is rotationally driven in the buckled state. Provision of the inner tube support tube 3d and the outer tube support tube 3c can suppress occurrence of such buckling.

The telescopic mechanism 3 has an inner flow path 3f formed on a radially inner side of the inner tube support tube 3d and the outer tube support tube 3c in the fully retracted state, and the inner flow path 3f allows priming liquid to flow from a rear end portion of the telescopic mechanism 3 to a front end portion of the telescopic mechanism 3 in the fully retracted state.

The telescopic mechanism 3 has an outer tube spacer 3g that is located between the proximal outer tube 3a and the outer tube support tube 3c and holds the outer tube support tube 3c, an outer peripheral surface of the outer tube spacer 3g is in contact with an inner peripheral surface of a front end portion of the proximal outer tube 3a, an inner peripheral surface of the outer tube spacer 3g is in contact with an outer peripheral surface of a front end portion of the outer tube support tube 3c, and the outer tube spacer 3g forms an outer tube communication flow path (not shown) that communicates the outer flow path 3e with the inner flow path 3f.

The telescopic mechanism 3 has an inner tube spacer 3h that is located between the proximal inner tube 3b and the inner tube support tube 3d and holds the inner tube support tube 3d, an outer peripheral surface of the inner tube spacer 3h is in contact with an inner peripheral surface of a rear end portion of the proximal inner tube 3b, an inner peripheral surface of the inner tube spacer 3h is in contact with an outer peripheral surface of a rear end portion of the inner tube support tube 3d, and the inner tube spacer 3h forms an inner tube communication flow path (not shown) that communicates the outer flow path 3e with the inner flow path 3f.

The image diagnosis catheter 1 has a relay connector 5 that connects the sheath 2 and a distal end portion of the telescopic mechanism 3 (a distal end portion of the proximal outer tube 3a), and a hub 6 connected to a proximal end portion of the telescopic mechanism 3 (a proximal end portion of the proximal inner tube 3b), and the hub 6 has a port 6a to which an injection device (not shown) for priming liquid is connected and an internal flow path 6b that communicates a flow path in the injection device connected to the port 6a with the inner flow path 3f and the outer flow path 3e of the telescopic mechanism 3. The drive shaft 4 extends through the internal flow path 6b of the hub 6 and has, at a proximal end portion of the drive shaft 4, a connection portion (not shown) to an external device.

Priming is performed by injecting priming liquid from the port 6a by the injection device, causing the priming liquid to flow in this order through the internal flow path 6b of the hub 6, the inner flow path 3f and the outer flow path 3e of the telescopic mechanism 3, and a flow path in the sheath 2, and discharging the priming liquid from a distal end opening (not shown) of the sheath 2.

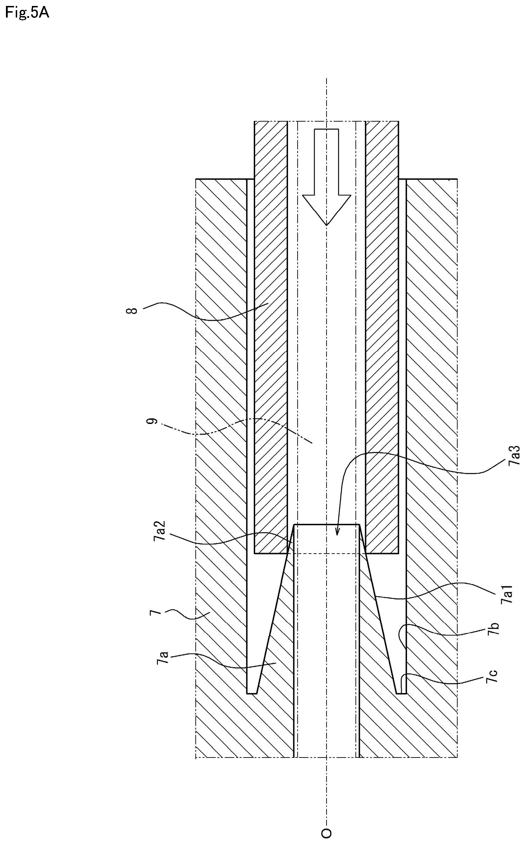

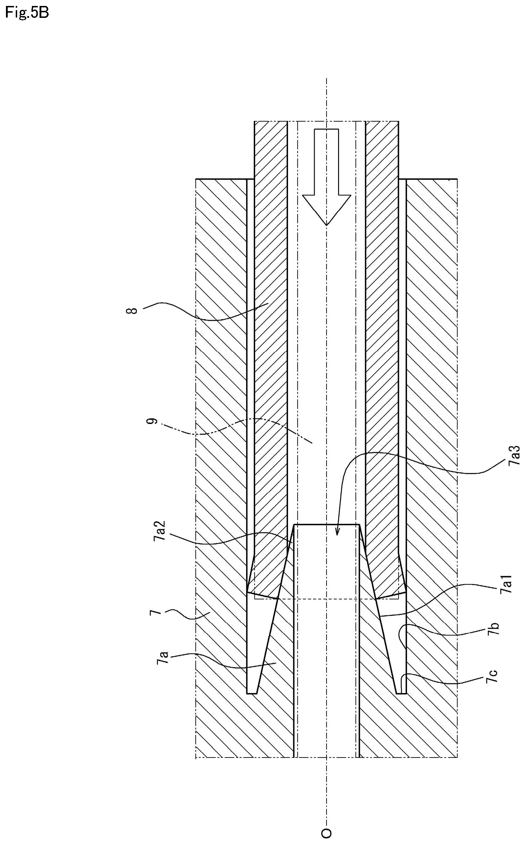

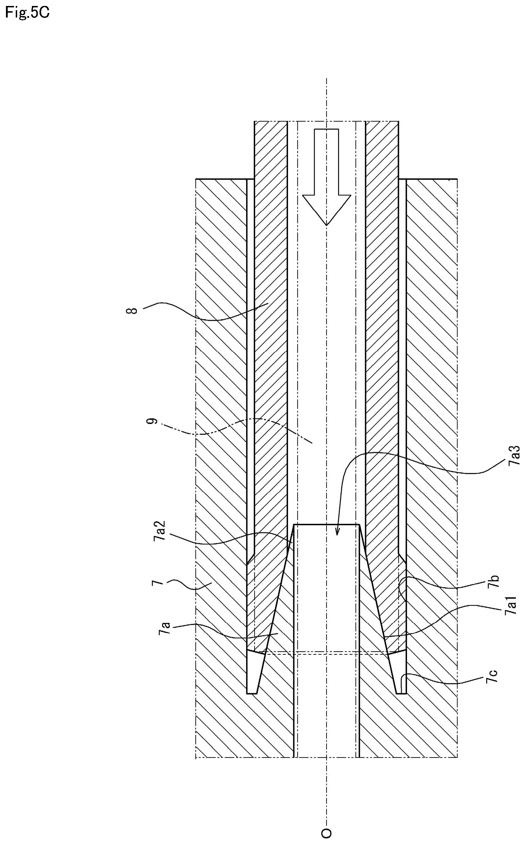

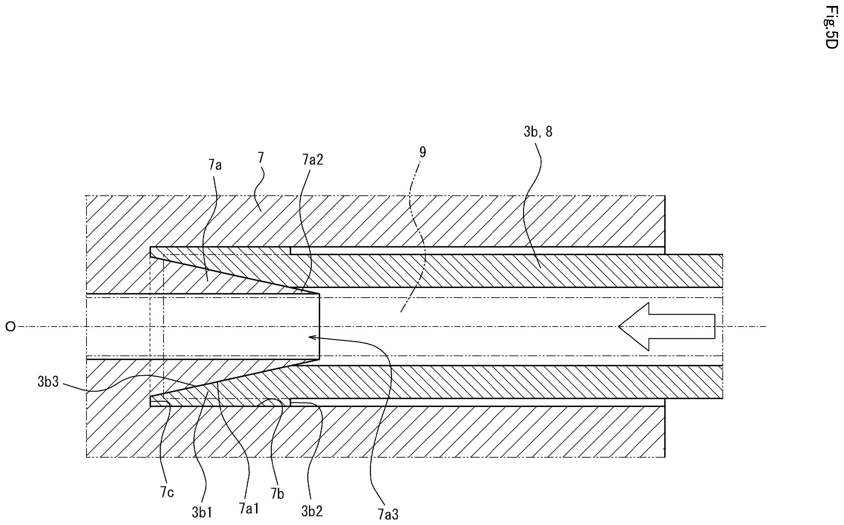

As shown in Figs. 5A to 5D, the image diagnosis catheter 1 may be manufactured by an image diagnosis catheter manufacturing method including a proximal inner tube 3b forming step of forming the guide surface 3b3 and the enlarged-diameter portion 3b1 by using a mold 7 having a guide surface molding surface 7a1 having a shape along the guide surface 3b3 and an enlarged-diameter portion molding surface 7b having a shape along an outer peripheral surface of the enlarged-diameter portion 3b1, and bringing a front end portion in a molten state of a tubular body 8, which is a preformed product of the proximal inner tube 3b, into contact with the guide surface molding surface 7a1 and the enlarged-diameter portion molding surface 7b to deform the front end portion. According to the above configuration, the guide surface 3b3 and the enlarged-diameter portion 3b1 of the proximal inner tube 3b can be formed simultaneously, so efficient manufacturing can be achieved.

The enlarged-diameter portion molding surface 7b has a constant diameter from a front end portion to a rear end portion, an outer diameter of the tubular body 8 is smaller than the diameter of the enlarged-diameter portion molding surface 7b, and a diameter of a front end of the guide surface molding surface 7a1, which extends rearward while gradually decreasing in diameter, is larger than an inner diameter of a front end portion of the tubular body 8. According to the above configuration, by advancing the tubular body 8 relative to the mold 7 due to an external force as shown by the white outlined arrows in Figs. 5A to 5D, a front end portion of the tubular body 8 is pressed against the guide surface molding surface 7a1 and is deformed to have an enlarged diameter, and the front end portion is brought into close contact with the enlarged-diameter portion molding surface 7b, so that the guide surface 3b3 and the enlarged-diameter portion 3b1 of the proximal inner tube 3b can be formed simultaneously. In the present embodiment, a front end surface forming surface 7c having a shape along the front end surface of the proximal inner tube 3b is provided between the front end of the guide surface molding surface 7a1 and the front end of the enlarged-diameter portion molding surface 7b, but the present disclosure is not limited thereto, and the shape or presence or absence of the front end surface forming surface 7c can be set as appropriate in accordance with the shape or presence or absence of the front end surface of the proximal inner tube 3b.

The external force is not particularly limited, and may be, for example, motive power of a machine or the like, or may be gravity. In order to advance the tubular body 8 relative to the mold 7, the external force may be applied only to the mold 7, may be applied only to the tubular body 8, or may be applied to both. The mold 7 is not limited to a configuration integrally having the guide surface molding surface 7a1 and the enlarged-diameter portion molding surface 7b as illustrated, and, for example, although not shown, the mold 7 may have a configuration in which the guide surface molding surface 7a1 and the enlarged-diameter portion molding surface 7b have movable portions capable of relative movement with respect to each other in the proximal inner tube 3b forming step.

The mold 7 has a projection 7a having the guide surface molding surface 7a1 on an outer peripheral surface, the projection 7a has an insertion portion 7a2 located rearward of the guide surface molding surface 7a1, and an outer peripheral surface of the insertion portion 7a2 extends rearward while gradually decreasing in diameter from a rear end of the guide surface molding surface 7a1. According to the above configuration, even when the tubular body 8 is eccentric relative to the guide surface molding surface 7a1 when the tubular body 8 is advanced relative to the mold 7 in the proximal inner tube 3b forming step, an inner peripheral surface of a front end portion of the tubular body 8 can be guided by the insertion portion 7a2 to the guide surface molding surface 7a1, so stabilization of manufacturing can be achieved.

In the proximal inner tube 3b forming step, a front end portion of the tubular body 8 is heated through the heated mold 7 so as to be brought into a molten state.

According to the above configuration, the front end portion of the tubular body 8 can be deformed while being melted by contact with the mold 7, so a further increase in manufacturing efficiency can be achieved. A method for heating the mold 7 is not particularly limited, and, for example, high-frequency induction heating can be used. The proximal inner tube 3b forming step may have a configuration in which the tubular body 8 is directly heated without using the mold 7.

The projection 7a has a hole 7a3 extending frontward from a rear end of the projection 7a, and the image diagnosis catheter manufacturing method may have a configuration in which, in the proximal inner tube 3b forming step, in a state in which a linear member 9 is inserted into the hole 7a3 of the projection 7a and the tubular body 8, the tubular body 8 is advanced relative to the mold 7 until the tubular body 8 comes into contact with the projection 7a. According to the above configuration, in the proximal inner tube 3b forming step, the tubular body 8 can be easily guided with high accuracy relative to the mold 7 until the tubular body 8 comes into contact with the projection 7a. The hole 7a3 of the projection 7a can be formed as a through hole or a bottomed hole.

The image diagnosis catheter manufacturing method may have a configuration in which, in the proximal inner tube 3b forming step, in a state in which the linear member 9 is inserted into the hole 7a3 of the projection 7a and the tubular body 8, the tubular body 8 is advanced relative to the mold 7 at least from a time when the tubular body 8 contacts the projection 7a until the guide surface 3b3 and the enlarged-diameter portion 3b1 can be formed. According to the above configuration, in the proximal inner tube 3b forming step, the tubular body 8 can be easily guided with high accuracy relative to the mold 7 at least from a time when the tubular body 8 contacts the projection 7a until the guide surface 3b3 and the enlarged-diameter portion 3b1 can be formed.

Although the embodiment of the present disclosure has been described above, the present disclosure is not limited to the embodiment described above, and the embodiment described above can be modified in various ways without departing from the gist of the present disclosure.

### Reference Signs List

- 1: Image diagnosis catheter
- 2: Sheath
- 2a: Guide wire insertion member
- 3: Telescopic mechanism
- 3a: Proximal outer tube
- 3a1: Step surface
- 3a2: Proximal outer tube body
- 3a3: Unit connector
- 3b: Proximal inner tube
- 3b1: Enlarged-diameter portion
- 3b2: Stopper
- 3b3: Guide surface
- 3c: Outer tube support tube
- 3d: Inner tube support tube
- 3e: Outer flow path
- 3f: Inner flow path
- 3g: Outer tube spacer
- 3h: Inner tube spacer
- 4: Drive shaft
- 4a: Signal transmitter and receiver
- 5: Relay connector
- 6: Hub
- 6a: Port
- 6b: Internal flow path
- 7: Mold
- 7a: Projection
- 7a1: Guide surface molding surface
- 7a2: Insertion portion
- 7a3: Hole
- 7b: Enlarged-diameter portion molding surface
- 7c: Front end surface forming surface
- 8: Tubular body
- 9: Linear member
- ∘: Central axis line
- θ: Inclination angle

## Claims

1. An image diagnosis catheter comprising: a proximal outer tube; a proximal inner tube; an outer tube support tube; and a drive shaft,
wherein, in a fully advanced state in which the proximal inner tube is pushed farthest into the proximal outer tube, the outer tube support tube, the proximal inner tube, and the proximal outer tube form a multiple tube structure located in an order of the outer tube support tube, the proximal inner tube, and the proximal outer tube toward a radially outer side from the drive shaft,
in a fully retracted state in which the proximal inner tube is drawn out farthest from the proximal outer tube, a rear end of the outer tube support tube is located on a front side of a front end of the proximal inner tube,
an outer peripheral surface of a front end portion of the proximal inner tube includes an enlarged-diameter portion having a rear end portion as a stopper that abuts against the proximal outer tube when the proximal inner tube is drawn out from the proximal outer tube and reaches the fully retracted state,
an inner peripheral surface of the front end portion of the proximal inner tube includes a guide surface extending rearward while gradually decreasing in diameter from a front end surface of the proximal inner tube, and
the guide surface is configured to guide a rear end portion of the outer tube support tube when the proximal inner tube is pushed into the proximal outer tube from the fully retracted state.

2. The image diagnosis catheter according to claim 1, wherein an outer diameter of the enlarged-diameter portion is constant from a front end portion to the rear end portion.

3. The image diagnosis catheter according to claim 1, comprising:
an inner tube support tube,
wherein, in the fully advanced state, the inner tube support tube, the outer tube support tube, the proximal inner tube, and the proximal outer tube form the multiple tube structure located in an order of the inner tube support tube, the outer tube support tube, the proximal inner tube, and the proximal outer tube toward the radially outer side from the drive shaft, and
in the fully retracted state, a front end of the inner tube support tube is located on the front side of the rear end of the outer tube support tube.

4. The image diagnosis catheter according to claim 3, wherein a telescopic mechanism formed of the proximal outer tube, the proximal inner tube, the outer tube support tube, and the inner tube support tube includes an outer flow path formed between the proximal inner tube and the proximal outer tube, and the inner tube support tube and the outer tube support tube in the fully retracted state, and the outer flow path allows priming liquid to flow from a rear end portion of the telescopic mechanism to a front end portion of the telescopic mechanism in the fully retracted state.

5. An image diagnosis catheter manufacturing method for manufacturing the image diagnosis catheter according to claims 1 to 4, the method comprising:
a proximal inner tube forming step of forming the guide surface and the enlarged-diameter portion by using a mold including a guide surface molding surface having a shape along the guide surface and an enlarged-diameter portion molding surface having a shape along an outer peripheral surface of the enlarged-diameter portion, and bringing a front end portion in a molten state of a tubular body that is a preformed product of the proximal inner tube into contact with the guide surface molding surface and the enlarged-diameter portion molding surface to deform the front end portion.

6. The image diagnosis catheter manufacturing method according to claim 5, wherein the enlarged-diameter portion molding surface has a constant diameter from a front end portion to a rear end portion, an outer diameter of the tubular body is smaller than the diameter of the enlarged-diameter portion molding surface, and a diameter of a front end of the guide surface molding surface extending rearward while gradually decreasing in diameter is larger than an inner diameter of a front end portion of the tubular body.
